(19) Europäisches Patentamt
European Patent Office
Office européen des brevets

(11) Veröffentlichungsnummer : **0 068 371**
**B1**

# (12) EUROPÄISCHE PATENTSCHRIFT

(45) Veröffentlichungstag der Patentschrift : **21.03.84**

(51) Int. Cl.³ : **C 07 C149/10**, C 07 C149/14, C 07 D333/08

(21) Anmeldenummer : **82105399.8**

(22) Anmeldetag : **19.06.82**

## (54) Verfahren zur Herstellung von Sulfiden.

(30) Priorität : **01.07.81 DE 3125920**

(43) Veröffentlichungstag der Anmeldung : **05.01.83 Patentblatt 83/01**

(45) Bekanntmachung des Hinweises auf die Patenterteilung : **21.03.84 Patentblatt 84/12**

(84) Benannte Vertragsstaaten : **BE DE FR GB**

(56) Entgegenhaltungen :
**SYNTHESIS, Nr. 7, Juli 1978, Seite 452 J. DRABOWICZ et al.: "An iodine-catalysed reduction of sulphoxides by formamidinesulphinic acid"**
**CHEMICAL ABSTRACTS, Band 79, Nr. 5, 6. August 1973, Seite 420, Nr. 31620n, Columbus, Ohio, USA TSE.LOK HO et al.: "Deoxygenation of sulfoxides" & SYNTHESIS 1973, (4), 206-207**

(73) Patentinhaber : **BASF Aktiengesellschaft**
**Carl-Bosch-Strasse 38**
**D-6700 Ludwigshafen (DE)**

(72) Erfinder : **Lardon, Hartmut, Dr.**
**Bruesseler Ring 2**
**D-6700 Ludwigshafen (DE)**
Erfinder : **Seybold, Guenther, Dr.**
**Friedrich-Ebert-Strasse 14**
**D-6701 Neuhofen (DE)**

EP 0 068 371 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Sulfiden

Die Erfindung betrifft ein Verfahren zur Herstellung von Sulfiden durch Umsetzung von Sulfoxiden mit Hydrazin- oder Hydroxylaminderivaten oder ihren quartären Salzen.

In Org. Prep. Proc. Int. *9*, 64-83 (1977) werden zahlreiche Methoden zur Reduktion von Sulfoxiden zu Sulfiden beschrieben. Die bekannten Verfahren liefern jedoch entweder mäßige Ausbeuten oder gehen von teuren bzw. schwer zugänglichen Chemikalien aus.

Es wurde nun gefunden, daß man Sulfide der Formel

$$R^1{-}S{-}R^2 \qquad (I)$$

worin die Reste $R^1$ und $R^2$ gleich oder verschieden sein können und jeweils einen aliphatischen, cycloaliphatischen, araliphatischen oder aromatischen Rest bedeuten oder zusammen mit dem benachbarten Schwefelatom auch Glieder eines heterocyclischen Ringes bezeichnen, durch Reduktion von Sulfoxiden vorteilhaft erhält, wenn man Sulfoxide der Formel

$$R^1{-}\overset{\overset{O}{\parallel}}{S}{-}R^2 \qquad (II)$$

worin $R^1$ und $R^2$ die vorgenannte Bedeutung besitzen, mit Aminderivaten der Formel

$$H_2N{-}XR^3 \qquad (III)$$

worin X ein Sauerstoffatom oder den Rest

$$\overset{H}{\underset{|}{}}\!\!{-}N{-}$$

bedeutet, $R^3$ ein Wasserstoffatom, eine Sulfonsäuregruppe oder einen Acylrest bezeichnet, oder ihren Ammoniumsalzen umsetzt, wobei die in $R^1$ und $R^2$ gegebenenfalls vorhandenen Carbonylgruppen im Falle der Bedeutung von $R^3$ als Wasserstoff und von X als Sauerstoff zu Ketoximen und im Falle der Bedeutung von $R^3$ als Wasserstoff und von X als Rest

$$\underset{H}{{-}N{-}}$$

zu Hydrazonen umgesetzt werden.

Die Umsetzung kann für den Fall der Verwendung von Hydroxylamin-o-sulfonsäure und Dimethylsulfoxid durch die folgenden Formeln wiedergegeben werden :

$$2\ H_2N{-}O{-}SO_3H + H_3C{-}\overset{\overset{O}{\parallel}}{S}{-}CH_3 \xrightarrow[-H_2O]{-N_2} H_3C{-}S{-}CH_3 + 2H_2SO_4\ .$$

Im Vergleich zu den bekannten Verfahren geht das Verfahren von einfacheren und wirtschaftlicheren Ausgangsstoffen aus und liefert auf einfacherem und wirtschaftlicherem Wege eine große Zahl von Sulfiden in guter Ausbeute und Reinheit. Alle diese vorteilhaften Eigenschaften sind im Hinblick auf den Stand der Technik überraschend.

Die Ausgangsstoffe II und III können miteinander im stöchiometrischen Verhältnis oder im Überschuß, zweckmäßig im Verhältnis von 1 bis 4, vorzugsweise 2 bis 3 Äquivalenten Ausgangsstoff III oder ihrem Ammoniumsalz je Mol Ausgangsstoff II umgesetzt werden. Bevorzugte Ausgangsstoffe II und dementsprechend bevorzugte Endstoffe I sind solche, in deren Formeln die Reste $R^1$ und $R^2$ gleich oder verschieden sein können und jeweils einen Alkylrest mit 1-8 Kohlenstoffatomen, einen Cycloalkylrest mit 5 bis 8 Kohlenstoffatomen, einen Aralkylrest oder Alkylarylrest mit 7-12 Kohlenstoffatomen, einen Phenylrest bedeuten oder zusammen mit dem benachbarten Schwefelatom auch einen heterocyclischen 5- oder 6-gliedrigen Ring bezeichnen. Die vorgenannten Reste können noch durch unter den Reaktionsbedingungen inerte Gruppen, z. B. Oxogruppen, Cyano-, Alkyl-, Alkoxigruppen mit jeweils 1-7 C-Atomen, substituiert sein. Ist $R^1$ und/oder gewünschtenfalls $R^2$ jeweils ein Keton und $R^3$ ein Wasserstoffatom, so wird neben der erfindungsgemäßen Reaktion die Ketogruppe im Falle X = 0 in die Ketoximgruppe bzw. im Falle X = NH in die Hydrazongruppe umgewandelt.

So sind z. B. folgende Sulfoxide als Ausgangsstoffe II geeignet : Dimethyl-, Dipropyl-, Diisopropyl-, Dibutyl-, Diisobutyl-, Di-sek.-butyl ; Di-tert.-butyl-, Dicyclohexyl-, Dicyclopentyl-, Dibenzyl-, Diphenyl-, Dio-methylphenyl-, Di-m-methylphenyl-, Di-p-methylphenyl-, Tetramethylen-, Pentamethylen-sulfoxid ; 3,3-Dimethyl-1-methylsulfinyl-2-butanon, 2-Methylsulfinylacetophenon, 4'-Methoxi-2-methyl-sulfinylacetophenon, 4'-Methyl-2-methylsulfinylacetophenon, 4'-Nitro-2-methylsulfinylacetophenon, 4'-Chlor-2-methylsulfinylacetophenon, 1-Methylsulfinyl-2-propanon, 1-Methylsulfinyl-3-butanon.

Ausgangsstoff III als Hydrazin oder Hydroxylamin kann als substituierte Verbindung verwendet werden, in der Regel wird man aber unsubstituierte Hydrazine bzw. Hydroxylamine verwenden. Die Substituenten können die Sulfonsäuregruppe oder die Acylgruppe sein.

Die Acylreste des Ausgangsstoffs III können aliphatische, cycloaliphatische, araliphatische oder aromatische Reste bedeuten. Im einzelnen seien z. B. die folgenden Acyle genannt : Methyl-, Ethyl-, Propyl-, Isopropyl-, Butyl-, Isobutyl-, sec.-Butyl-, tert.-Butyl-, Cyclohexyl-, Phenyl-, methyl-, Phenylcarbonyl-gruppe.

Das Hydroxylamin bzw. Hydrazin wird vorteilhaft in Form seiner Salze verwendet. Geeignete Salze sind z. B. das Chlorid, Nitrat, Sulfat, Formiat oder Acetat des Hydroxylamins bzw. Hydrazins. Handelt es sich bei den Ausgangsstoffen II um β-Ketosulfoxide, so arbeitet man in der Regel bei pH 6 bis 8, vorzugsweise pH 7.

Die Umsetzung kann vorteilhaft bei einer Temperatur von 20 bis 150 °C, vorzugsweise 55 bis 145 °C, drucklos oder unter Druck, kontinuierlich oder diskontinuierlich durchgeführt werden.

Die Umsetzung kann mit oder ohne ein Lösungsmittel durchgeführt werden. Zweckmäßig verwendet man unter den Reaktionsbedingungen inerte Lösungsmittel. Als Lösungsmittel kommen z. B. in Frage : Ether, z. B. Ethylpropylether, Methyltert.-butylether, n-Butylethylether, Di-n-butylether, Diisobutylether, Diisoamylether, Diisopropylether, Cyclohexylmethylether, Diethylether, Ethylenglykoldimethylether, Tetrahydrofuran, Dioxan ; Ester wie Methylacetat, n-Propylacetat, Methylpropionat, Butylacetat, Ethylformiat, Phthalsäuremethylester, Benzoesäuremethylester, Essigester, Phenylacetat ; Alkanole und Cycloalkanole wie Ethanol, Methanol, n-Butanol, Isobutanol, tert.-Butanol, Glykol, Glycerin, n-Propanol, Isopropanol, Amylalkohol, Cyclohexanol, 2-Methyl-4-pentanol, Ethylenglykolmonoethylether, 2-Ethylhexanol, Methylglykol, n-Hexanol, Isohexylalkohol, Isoheptylalkohol, n-Heptanol, Ethylbutanol, Nonylalkohol, Dodecylalkohol, Methylcyclohexanol, insbesondere solche mit 1 bis 4 Kohlenstoffatomen, substituierte Amide wie Dimethylformamid ; Wasser ; Halogenkohlenwasserstoffe, insbesondere Chlorkohlenwasserstoffe, z. B. Tetrachlorethylen, 1,1,2,2- oder 1,1,1,2-Tetrachlorethan, Amylchlorid, Dichlorpropan, Methylchlorid, Dichlorbutan, Chloroform, Tetrachlorkohlenstoff, 1,1,1-oder 1,1,2-Trichlorethan, Trichlorethylen, Pentachlorethan, 1,2-Dichlorethan, 1,1-Dichlorethan, n-Propylchlorid, 1,2-cis-Dichlorethylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol. Bevorzugt sind Ethanol, Isobutanol, Dimethylformamid, Tetrahydrofuran, Dimethoxiethan und n-Butanol ; und entsprechende Gemische. Zweckmäßig verwendet man das Lösungsmittel in einer Menge von 100 bis 10 000 Gewichtsprozent, vorzugsweise von 100 bis 2 000 Gewichtsprozent, bezogen auf Ausgangsstoff II. Vorteilhaft sind Gemische mit Wasser und z. B. Alkanolen, wobei zweckmäßig die Wassermenge 0 bis 80 Gewichtsprozent, bezogen auf das Gesamtgemisch, gewählt wird.

Die Reaktion kann wie folgt durchgeführt werden : Ein Gemisch der Ausgangsstoffe II und III (oder quartäres Salz) wird bei vorgenannter Temperatur während 1 bis 32, vorzugsweise 1 bis 7 Stunden gehalten. Dann wird der Endstoff in üblicher Weise, z. B. durch Destillation, Extraktion oder Filtration, abgetrennt.

Die nach dem Verfahren der Erfindung herstellbaren Sulfide sind wertvolle Ausgangsstoffe für die Herstellung von Pharmazeutika, Schädlingsbekämpfungsmitteln und Farbstoffen. Das Verfahren eignet sich besonders zur Herstellung von Ketoximsulfiden, die wertvolle Zwischenprodukte für Insektizide und andere Wirkstoffe darstellen. Die dafür als Ausgangsprodukte verwendeten Ketosulfoxide werden nach der erfindungsgemäßen Methode in einem Schritt reduziert und oximiert.

Bezüglich der Verwendung wird auf die vorgenannte Veröffentlichung verwiesen.

Die in den Beispielen genannten Teile sind Gewichtsteile.

## Beispiel 1

Zu 64,8 Teilen Hydroxylammoniumsulfat gibt man bei 120 °C innerhalb einer Stunde 26 Teile Dimethylsulfoxid und läßt eine Stunde lang bei der gleichen Reaktionstemperatur nachrühren. Bereits während der Zugabe beginnt das gebildete Dimethylsulfid in eine gekühlte Vorlage abzudestillieren. Nach beendeter Umsetzung wird das Destillat nochmals destilliert. Bei 37 °C gehen 15,9 Teile (77 % der Theorie) Dimethylsulfid über.

## Beispiel 2

Zu 22,6 Teilen Hydroxylamin-O-sulfonsäure gibt man langsam bei 70 °C innerhalb von 10 Minuten 4,6 Teile Dimethylsulfoxid und läßt eine Stunde lang bei 100 °C nachrühren. Dabei werden 2,2 Teile Dimethylsulfid (60 % der Theorie) vom Kp 37 °C isoliert.

Beispiel 3

Analog Beispiel 1 werden 70 Teile Hydroxylammoniumchlorid zunächst in 50 Teilen n-Butanol suspendiert und danach bei 120 °C mit einer Lösung von 39 Teilen Dimethylsulfoxid in 80 Teilen n-Butanol versetzt. Man erhält 23,5 Teile Dimethylsulfid (76 % der Theorie) vom Kp 37 °C.

Beispiel 4

Zu 52,5 Teilen Hydrazindihydrochlorid gibt man bei 60 °C innerhalb von 2 Stunden 78 Teile Dimethylsulfoxid und läßt bei 90 °C eine Stunde lang nachrühren. Bereits während der Zugabe beginnt das gebildete Dimethylsulfid in eine gekühlte Vorlage abzudestillieren. Nach beendeter Umsetzung wird das Destillat nochmals destilliert. Bei 37 °C gehen 60,9 Teile (98 % der Theorie) Dimethylsulfid über.

Beispiele 5 bis 10

Analog zu Beispiel 1 bzw. 4 werden sowohl mit Hydrazinen III (als Salz) sowie Hydroxylaminen III (als Salz) folgende Sulfoxide reduziert. Das Reaktionsgemisch wird mit 100 Teilen Wasser versetzt und mit je 100 Teilen Chloroform dreimal extrahiert. Der Extrakt wird destilliert.

| Beispiel Nr. | Ausgangsstoff | Ausbeute des Endstoffs in % der Theorie mit Hydroxylamin III | mit Hydrazin III | kp des Endstoffs in °C/mbar |
|---|---|---|---|---|
| 5 | Di-n-propylsulfoxid | 71 | 93 | 142-143/1 000 |
| 6 | Di-iso-propylsulfoxid | 76 | 91 | 120-121/1 000 |
| 7 | Di-n-butylsulfoxid | 80 | 95 | 85-86 /20 |
| 8 | Di-tert.-butylsulfoxid | 70 | 89 | 40-41 /20 |
| 9 | Tetramethylensulfoxid | 72 | 92 | 119-120/1 000 |
| 10 | Dibenzylsulfoxid | 70 | 90 | 130-131/0,45 |

Die gebildeten Sulfide werden durch Vergleich mit den spektroskopischen und physikalischen Daten authentischer Proben charakterisiert.

Beispiel 11

56,4 Teile 3,3-Dimethyl-1-methylsulfinyl-2-butanon, 128,6 Teile Hydroxylammoniumchlorid, 98,6 Teile Natriumcarbonat, 230 Teile Wasser sowie 400 Teile iso-Butanol bei pH 7 werden 32 Stunden lang bei 95 °C gerührt. Nach dem Abkühlen auf Raumtemperatur wird die wäßrige Phase verworfen und die organische Phase fraktioniert destilliert. Bei 85-86 °C/0,9 mbar gehen 47,9 Teile (85 % der Theorie) 3,3-Dimethyl-1-methylthio-2-butanonoxim mit dem Brechungsindex $n_D^{24}$ = 1,500 8 über.

| | C | H | O | N | S |
|---|---|---|---|---|---|
| ber.: | 52,1 | 9,4 | 9,9 | 8,7 | 19,9 |
| gef.: | 53,5 | 9,5 | 10,0 | 8,5 | 19,2 |

Beispiel 12

18,2 Teile 2-Methylsulfinylacetophenon, 29,6 Teile Hydroxylammoniumsulfat, 19,2 Teile Natriumcarbonat, 80 Teile Wasser sowie 100 Teile Ethanol werden 17 Stunden lang unter Rückfluß erhitzt. Anschließend destilliert man die Lösungsmittel weitestgehend ab, gibt 300 Teile Wasser zu und extrahiert dreimal mit je 70 Teilen Essigester. Die vereinigten und über Natriumsulfat getrockneten organischen Phasen werden im Vakuum vom Lösungsmittel befreit und aus Cyclohexan umkristallisiert. Man erhält 8,6 Teile (48 % der Theorie) an 2-Methylthioacetophenonoxim mit dem Schmelzpunkt 59-60 °C.

Beispiel 13

Ein Gemisch aus 10,5 Teilen Methylphenylsulfoxid und 21 Teilen Hydroxylammoniumchlorid wird 5 Stunden lang bei 95 °C gerührt. Nach dem Abkühlen auf Raumtemperatur rührt man das Reaktionsgemisch mit 100 Teilen Methylenchlorid, filtriert unlösliche Bestandteile ab und wäscht diese mehrmals mit Methylenchlorid. Die vereinigten Filtrate werden im Vakuum eingeengt. Aus dem Rückstand destillieren bei 45 °C/0,8 mbar 7,0 Teile (74 % der Theorie) Methylphenylsulfid ab.

Beispiel 14

Ein Gemisch aus 15,15 Teilen Diphenylsulfoxid und 8,4 Teilen Hydrazindihydrochlorid wird 21 Stunden lang bei 140 °C gerührt. Nach dem Abkühlen auf Raumtemperatur versetzt man das Reaktionsgemisch mit 200 Teilen Wasser, extrahiert viermal mit je 70 Teilen Chloroform und trocknet die vereinigten organischen Phasen über Natriumsulfat. Nach dem Entfernen des Lösungsmittels destillieren bei 92-95 °C/0,1 mbar 9,9 Teile (71 % der Theorie) Diphenylsulfid ab.

Beispiele 15 und 16

Analog zu Beispiel 13 (Hydroxylamin) und Beispiel 14 (Hydrazin) werden folgende Sulfoxide nach beiden Methoden reduziert und statt durch Destillation durch Umkristallisation gereinigt :

| Beispiel Nr. | Ausgangsstoff | Fp des Endstoffs in °C | Ausbeute des Endstoffs in % der Theorie mit Hydroxylamin III (als Salz) | mit Hydrazin III (als Salz) |
|---|---|---|---|---|
| 15 | Bis-(4-chlorphenyl)-sulfoxid | 93 - 95 | 68 | 78 |
| 16 | Bis-(4-methylphenyl)-sulfoxid | 57 | 71 | 80 |

**Anspruch**

Verfahren zur Herstellung von Sulfiden der Formel

$$R^1—S—R^2 \qquad (I)$$

worin die Reste $R^1$ und $R^2$ gleich oder verschieden sein können und jeweils einen aliphatischen, cycloaliphatischen, araliphatischen oder aromatischen Rest bedeuten oder zusammen mit dem benachbarten Schwefelatom auch Glieder eines heterocyclischen Ringes bezeichnen, durch Reduktion von Sulfoxiden, dadurch gekennzeichnet, daß man Sulfoxide der Formel

$$R^1-\overset{\overset{O}{\|}}{S}-R^2 \qquad (II)$$

worin $R^1$ und $R^2$ die vorgenannte Bedeutung besitzen, mit Aminderivaten der Formel

$$H_2N—XR^3 \qquad (III)$$

worin X ein Sauerstoffatom oder den Rest

$$-\overset{\overset{H}{|}}{N}-$$

bedeutet, $R^3$ ein Wasserstoffatom, eine Sulfonsäuregruppe oder einen Acylrest bezeichnet, oder ihren Ammoniumsalzen umsetzt, wobei die in $R^1$ und $R^2$ gegebenenfalls vorhandenen Carbonylgruppen im Falle der Bedeutung von $R^3$ als Wasserstoff und von X als Sauerstoff zu Ketoximen und im Falle der Bedeutung von $R^3$ als Wasserstoff und von X als Rest

$$-\underset{\underset{H}{|}}{N}-$$

zu Hydrazonen umgesetzt werden.

**Claim**

A process for the preparation of sulfides of the formula

$$R^1—S—R^2 \qquad (I)$$

where $R^1$ and $R^2$ can be identical or different and each is an aliphatic, cycloaliphatic, araliphatic or aromatic radical, or $R^1$ and $R^2$ together with the adjacent sulfur atom are also members of a heterocyclic ring, by reduction of a sulfoxide, wherein a sulfoxide of the formula

$$R^1{-}\overset{\overset{\displaystyle O}{\|}}{S}{-}R^2 \qquad (II)$$

where $R^1$ and $R^2$ have the above meanings, is reacted with an amine derivative of the formula

$$H_2N{-}XR^3 \qquad (III)$$

where X is oxygen or the radical

$$\overset{\overset{\displaystyle H}{|}}{-N-}$$

and $R^3$ is hydrogen, a sulfonic acid group or an acyl radical, or with its ammonium salt, the carbonyl groups which may be present in $R^1$ and $R^2$ being converted to ketoximes in the case in which $R^3$ is hydrogen and X is oxygen, and to hydrazones in the case in which $R^3$ is hydrogen and X is the radical

$$\underset{\underset{\displaystyle H}{|}}{-N-}$$

**Revendication**

Procédé de préparation de sulfures de la formule

$$R^1{-}S{-}R^2 \qquad (I)$$

dans laquelle les restes $R^1$ et $R^2$ peuvent être identiques ou différents et représenter chacun un groupe aliphatique, cycloaliphatique, araliphatique ou aromatique ou former avec l'atome de soufre adjacent des chaînons d'un noyau hétérocyclique, par réduction de sulfoxydes, caractérisé en ce que l'on fait réagir des sulfoxydes de la formule

$$R^1{-}\overset{\overset{\displaystyle O}{\|}}{S}{-}R^2 \qquad (II)$$

dans laquelle $R^1$ et $R^2$ possèdent les significations définies, avec des dérivés d'amine de la formule

$$H_2N{-}XR^3 \qquad (III)$$

dans laquelle X représente un atome d'oxygène ou un groupe

$$\overset{\overset{\displaystyle H}{|}}{-N-}$$

et $R^3$ désigne un atome d'hydrogène, un groupe acide sulfonique ou un reste acyle, ou avec des sels d'ammonium de tels dérivés, les groupes carbonyle éventuellement présents dans $R^1$ et $R^2$ étant transformés en cétoximes dans le cas où $R^3 = H$ et $X = 0$ et en hydrazones, lorsque $R^3 = H$ et $X =$

$$\underset{\underset{\displaystyle H}{|}}{-N-}$$